# EUROPÄISCHE PATENTSCHRIFT

(11) **EP 3 232 242 B1**
(45) Veröffentlichungstag und Bekanntmachung des Hinweises auf die Patenterteilung: **04.12.2019**
(21) Anmeldenummer: 16165215.1
(22) Anmeldetag: 14.04.2016
(51) Int. Cl.: G02B 7/00, F16M 11/04, A61B 90/20, F16M 11/22, A61B 90/25, F16M 11/42, G02B 21/00, B62B 5/00, B62B 5/04

(54) **MIKROSKOP MIT STATIVBREMSE**
MICROSCOPE WITH STAND BRAKE
MICROSCOPE COMPRENANT UN FREIN DE TREPIED

(43) Veröffentlichungstag der Anmeldung: 18.10.2017
(73) Patentinhaber: Leica Instruments (Singapore) Pte. Ltd., 608924 Singapore (SG)
(72) Erfinder: El Haddouchi, Hakim, 9436 Balgach (CH)
(74) Vertreter: Grunert, Marcus

(56) Entgegenhaltungen:
- DE-A1- 10 232 688
- DE-A1-102013 005 999
- DE-U1- 8 400 384
- DE-U1-202005 014 134
- US-A1- 2015 085 986

## Beschreibung

Die Erfindung betrifft ein Mikroskop mit einem Stativ, wobei das Stativ eine Bremseinrichtung zur Arretierung des Stativs aufweist.

Die vorliegende Erfindung soll im Folgenden ohne Beschränkung der Allgemeinheit bezogen auf Operationsmikroskope erläutert werden. Operationsmikroskope mit Bodenstativ weisen standardmäßig meist eine Fuß-Feststellbremse zur Arretierung des Stativs auf. Die Fußbremsen befinden sich an den Rollen des Stativs, auch Mikroskopfuß genannt. Die Fuß-Feststellbremsen werden in der Regel mit den Füßen bedient, das heißt arretiert und gelöst. Die Fuß-Feststellbremsen sind nur aktiv, wenn sie betätigt werden, das heißt im Grundzustand ist keine Bremse aktiviert. Somit ist das Gerät im Grundzustand bewegbar und es muss eine aktive Betätigung der Feststellbremse erfolgen, um das Gerät zu arretieren.

Dieser Umstand kann sicherheitsproblematisch werden, etwa wenn eine äußere Kraft auf das Operationsmikroskop bzw. Stativ einwirkt oder wenn dieses auf einem nicht horizontal-ebenen Untergrund steht. Es besteht dann die Wahrscheinlichkeit, dass das gesamte Gerät eigenständig in Bewegung gerät, wenn die Feststellbremse noch nicht aktiv betätigt wurde oder wenn vergessen wurde, diese zu betätigen.

Ein weiterer Nachteil der genannten Fuß-Feststellbremse besteht darin, dass diese je nach Gerätepositionierung sich in einer ungünstigen oder gar unerreichbaren Lage befinden kann, wodurch die Zugänglichkeit der Bremse eingeschränkt wird. Wird beispielsweise das Operationsmikroskop nahe an einen Operationstisch geschoben, kann der Mikroskopfuß unter dem Operationstisch verschwinden. Die Bremse befindet sich dann unter dem Tisch und kann gegebenenfalls nicht oder nur schwer betätigt oder gelöst werden. Schließlich gab es Fälle, in denen sich Fuß-Feststellbremsen ohne aktive Einwirkung gelöst haben, wodurch das Operationsmikroskop bei Einwirken von äußeren Kräften (Betätigung durch Bedienpersonal oder schräge/abschüssige Ebenen) bewegt werden und somit Schaden verursachen konnte.

Aus der DE 102 32 688 A1 ist ein Operationsmikroskop mit Stativ bekannt, bei dem ein Mundschalter vorgesehen ist, um eine Bremsvorrichtung zu betätigen, sodass eine Verlagerung des Mikroskops ermöglicht wird .Die Bremse wird nur bei Betätigung des Mundschalters, was einer Totmannbremse entspricht. Die Bremse wirkt dabei auf Gelenke zwischen zwei Armen des Stativs.

Aus der DE 84 00 384 U1 ist ein Bodenstativ für Stereomikroskope bekannt, wobei das Bodenstativ Rollen aufweist. Dabei ist eine Arretierungsvorrichtung vorgesehen, mittels welcher bei Verstellen eine Griffstange entweder Stützelemente, die das Stativ am Boden festhalten, angehoben werden können oder aber die Rollen ausgefahren werden können, wodurch eine Bewegung des Stativs ermöglicht wird. Diese Griffstange wird dabei mittels einer Klinke festgestellt.

Der vorliegenden Erfindung liegt daher die Aufgabe zugrunde, eine Bremseinrichtung für ein Mikroskopstativ, insbesondere ein Operationsmikroskopstativ, anzugeben, die die oben genannten Nachteile möglichst vermeidet. Insbesondere soll die Bremseinrichtung von einer Bedienperson leicht bedient werden können.

Diese Aufgabe wird durch ein Mikroskop gemäß Anspruch 1 gelöst. Vorteilhafte Ausgestaltungen ergeben sich aus den Unteransprüchen und der nachfolgenden Beschreibung.

Erfindungsgemäß ist die Mikroskopstativbremseinrichtung als Totmannbremse ausgeführt, so dass eine Nichtbetätigung der Bremseinrichtung das Stativ arretiert. Die bisher vorhandene Fuß-Feststellbremse kann erfindungsgemäß ganz ersetzt oder aber als zusätzliche Sicherheitsbremse beibehalten werden. Totmannbremsen sind an sich aus dem Bereich der Gepäckwagen oder Kofferkulis bekannt. Eine Nichtbetätigung der Totmannbremse führt erfindungsgemäß zu einer Arretierung des Stativs bzw. hält eine vorhandene Arretierung aufrecht. Umgekehrt führt erst eine Betätigung der Totmannbremse zu einem Lösen der Bremsen, so dass das Stativ bewegt werden kann.

Aufbau und Wirkungsweise einer Totmannbremse bei einem Rollwagen sind aus der DE 20 2013 000 849 U1 bekannt. Zu Einzelheiten bezüglich Aufbau und Funktionsweise einer Totmannbremse sei ausdrücklich auf diese Schrift verwiesen.

Es ist vorteilhaft, wenn die Totmannbremse über wenigstens eine Bremsbedieneinheit verfügt, durch deren Betätigung ein Lösen der Totmannbremse, also insbesondere der Bremse(n) der Totmannbremse erfolgt. Umgekehrt erfolgt durch eine Nichtbetätigung der Bremsbedieneinheit(en) eine Aktivierung der Totmannbremse und somit eine Arretierung des Mikroskopstativs. Es kann vorteilhaft sein, wenn die Bremsbedieneinheit in mehrere Segmente, beispielsweise in zwei Teile unterteilt ist. Auf diese Weise kann noch besser ein unbeabsichtigtes Verschieben des Stativs vermieden werden, wenn nämlich ein Lösen der Totmannbremse erst erfolgt, wenn beide Teile der Bremsbedieneinheit betätigt werden Auf diese Weise kann auch eine "Lenkung" des Stativs verbessert werden, die dann voraussetzt, dass beispielsweise beide getrennte Teile der Bedieneinheit betätigt sein müssen, während das Stativ auf diese Weise gelenkt und verschoben wird.

Die Bremsbedieneinheit kann ein Bedienhebel, ein Bedienknopf und/oder ein Bedienschalter sein. Prinzipiell ist es möglich, dass die Bremsbedieneinheit vom Fuß einer Bedienperson betätigt werden kann, aus den Eingangs genannten Gründen hat sich jedoch als vorteilhaft erwiesen, wenn die Bremsbedieneinheit ein von Hand bedienbarer Bedienhebel, Bedienknopf und/oder Bedienschalter ist. Dies vermeidet insbesondere bei Operationsmikroskopen die erschwerte Bedienbarkeit einer Fußbremse, wenn sich der Mikroskopfuß unter oder an einem Operationstisch befindet.

Hierbei hat es sich als besonders vorteilhaft erwiesen, wenn die Bremsbedieneinheit als Handgriff des Stativs ausgebildet ist. In diesem Fall übernimmt die Bremsbedieneinheit zwei Funktionen: Zum Einen dient sie zum Bewegen des Stativs, indem sie den Handgriff des Stativs bildet. Zum Anderen dient sie zum Lösen oder Aktivieren der Stativbremse. Zum Bewegen des Stativs muss also der Handgriff des Stativs umfasst werden, gleichzeitig muss die Bremsbedieneinheit betätigt werden, indem beispielsweise der Handgriff nach unten oder nach oben gedrückt oder in eine bestimmte Richtung gezogen wird, um die Totmannbremse zu lösen. Hierzu ist der Handgriff entsprechend schwenkbar gelagert bzw. in eine Richtung ausziehbar ausgestaltet.

Es ist ebenso vorteilhaft, wenn die Bremsbedieneinheit an einem festen Handgriff des Stativs, insbesondere unter- oder oberhalb des Handgriffs ausgebildet ist. Insbesondere ist die Bremsbedieneinheit derart an dem Handgriff des Stativs angeordnet, dass die Bremsbedieneinheit und der Handgriff zusammen mit einer Hand betätigt werden können. Beispielsweise kann die Hand einer Bedienperson den Handgriff des Stativs umfassen und gleichzeitig die Bremsbedieneinheit, etwa durch Ziehen oder Drücken eines Bedienhebels, betätigen. Auch ein in der Nähe des Handgriffs angeordneter Bedienknopf oder Bedienschalter könnte zusammen mit dem Handgriff des Stativs mit einer einzigen Hand betätigt werden, um die Stativbremse zu lösen und anschließend das Stativ zu verfahren. In dem Moment, in dem keine Betätigung der Bremsbedieneinheit mehr erfolgt, ist die Stativbremse, also die Totmannbremse, wieder aktiv und das Stativ arretiert.

Die Totmannbremse des Mikroskopstativs weist neben der geschilderten Bremsbedieneinheit insbesondere eine Übertragungseinheit zur eigentlichen Bremse bzw. den eigentlichen Bremsen auf. Letztere ist bzw. sind beispielsweise an den Rollen des Stativs angeordnet, wobei eine Betätigung der Bremsbedieneinheit über die Übertragungseinheit eine Deaktivierung der Bremse(n) hervorruft. Es ist insbesondere zweckmäßig, wenn die Totmannbremse rein mechanisch ausgebildet ist. Die Übertragung einer Betätigung der Bremsbedieneinheit über die Übertragungseinheit auf die Bremse(n) erfolgt dann in rein mechanischer Wirkungsweise. Dies ermöglicht eine völlige Unabhängigkeit der Stativbremse von einer Stromversorgung. Alternativ kann die Totmannbremse aber auch elektronisch und/oder hydraulisch ausgebildet sein. In diesen Fällen ist eine Stromversorgung, etwa durch Netzanschluss, Batterie oder Akkus sicherzustellen. Es sind auch Mischformen von zwei oder drei der genannten Wirkungsweisen vorstellbar, insbesondere eine mechanische Wirkungsweise, die elektronisch oder hydraulisch unterstützt wird, etwa eine mechanische Bremsbedieneinheit, wobei die Übertragungseinheit und/oder die eigentliche Bremse(n) elektronisch oder hydraulisch zu einer Bremsverstärkung führen.

Mikroskopstative tragen nicht nur das Mikroskop selbst, sondern in der Regel weitere Hilfsvorrichtungen, wie Beleuchtungseinrichtungen, beispielsweise für Operationsmikroskope. Darüber hinaus enthalten sie Zusatzgeräte, um das Stativ klein und kompakt ausbilden zu können. Dies führt insgesamt zu einem hohen Gewicht des Mikroskopstativs, sodass das Verfahren der Stative einen hohen Kraftaufwand erfordert. Es ist daher vorteilhaft, wenn das Stativ Rollen zum Verfahren des Stativs aufweist, wobei eine Antriebseinheit zum Antreiben mindestens eine dieser Rollen vorgesehen ist. Eine solche im Stativ vorhandene Antriebseinheit kann insbesondere die mindestens eine Rolle unterstützend dann antreiben, wenn eine Bewegung bzw. ein Verfahren des Stativs erfolgt. Da hierzu erfindungsgemäß ein Lösen der Totmannbremse erforderlich ist, ist die Antriebseinheit mit Vorteil derart eingerichtet, dass sie die mindestens eine Stativrolle unterstützend antreibt, wenn ein Lösen der Totmannbremse erfolgt. Hierzu kann die Antriebseinheit mit der Übertragungseinheit der Totmannbremse in Wirkverbindung stehen, wobei die Übertragungseinheit die Antriebseinheit derart ansteuert, dass diese mindestens eine der Stativrollen unterstützend antreibt, wenn die Bremsbedieneinheit betätigt wird.

In dieser vorteilhaften Ausgestaltung wird folglich beim Lösen der Totmannbremse durch ein Betätigen der Bremsbedieneinheit(en) die Antriebseinheit, insbesondere ein Elektromotor, aktiviert, um insbesondere alle Stativrollen gleichmäßig unterstützend anzutreiben. Auf diese Weise kann das Stativ leichtgängig verfahren und manövriert werden. Sobald die Bremsbedieneinheit(en) nicht mehr betätigt wird (bzw. werden) erfolgt eine Aktivierung der Totmannbremse, wobei die Aktivierung der Bremse, insbesondere der einzelnen Bremsen an den Stativrollen, elektronisch/hydraulisch unterstützt werden kann. Dies ermöglicht insbesondere auch ein sanftes und zugleich effektives Abbremsen des in Bewegung befindlichen Stativs. Ein als Antriebseinheit vorhandener Elektromotor kann zur Unterstützung des Bremsvorgangs (Generatorbetrieb oder elektronische Bremsung) eingesetzt werden. Eine für die Totmannbremse vorhandene Stromversorgung kann auch als Stromversorgung der Antriebseinheit verwendet werden. Insbesondere kann ein Akkumulator auch durch entstehende Bremsenergie aufgeladen werden.

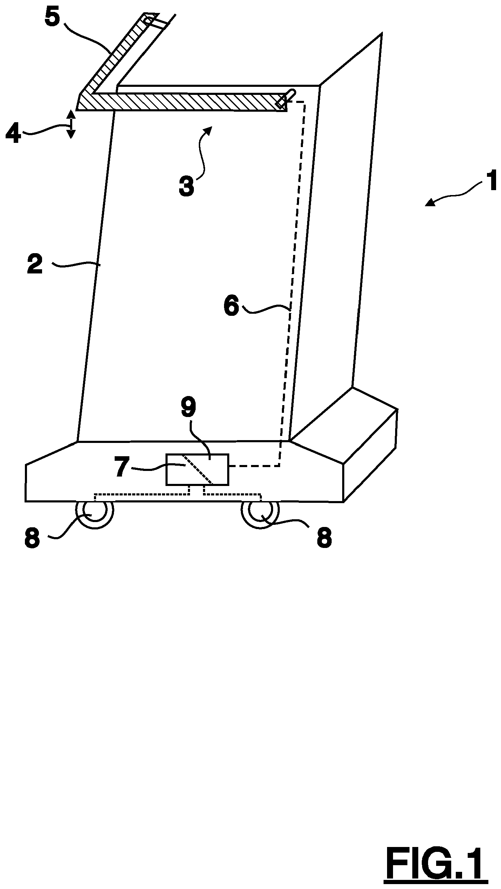

Weitere Vorteile und Ausgestaltungen der Erfindung ergeben sich aus der Beschreibung und der beiliegenden Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist anhand eines Ausführungsbeispieles in der Zeichnung schematisch dargestellt und wird im Folgenden unter Bezugnahme auf die Zeichnung beschrieben.

### Figurenbeschreibung

Die einzige Figur zeigt sehr schematisch ein erfindungsgemäßes Mikroskop mit Stativbremse.

In der Figur ist das erfindungsgemäße Mikroskop mit 1 bezeichnet. Das eigentliche Mikroskop ist, da es für die vorliegende Erfindung nicht relevant ist, nicht dargestellt. Das Stativ des Mikroskops 1 ist mit 2 bezeichnet. Zu sehen ist insbesondere der untere Teil des Stativs 2, der auch als Mikroskopfuß bezeichnet wird. Das Stativ 2 befindet sich auf Stativrollen 8, von denen die vorderen Rollen 8 schematisch dargestellt sind. Über einen Handgriff 5 ist das Stativ 2 über die Rollen 8 auf einem Boden bewegbar.

Im dargestellten Fall ist die Stativbremse als Totmannbremse 3 ausgeführt. Eine Nichtbetätigung der Totmannbremse 3 führt zu einer Arretierung des Stativs 2, während eine Betätigung der Totmannbremse 3 zu einem Lösen der Stativbremse mit der Möglichkeit der Bewegung des Stativs 2 führt. Die Bremsbedieneinheit 4 ist im vorliegenden Beispielsfall als Bedienhebel ausgeführt, der den Handgriff 5 des Stativs 2 bildet. Durch Herabdrücken des Bedienhebels/Handgriffs 5 wird die Totmannbremse 3 gelöst, so dass das Stativ 2 bewegt werden kann. Nach Betätigen bzw. Herabdrücken der Bremsbedieneinheit 4 bzw. nach Loslassen der Bremsbedieneinheit 4 kehrt diese wieder in ihre Ausgangslage zurück. Dazu ist die Bremsbedieneinheit bzw. der Handgriff 5 beispielsweise durch eine entsprechende Vorspannfederung in eine bestimmte Ausgangsposition positioniert. Durch Herabdrücken der Bremsbedieneinheit 4 bzw. des Handgriffs 5 gegen eine Federkraft kann die Totmannbremse 3 gelöst werden.

Im dargestellten Fall umfasst die Totmannbremse 3 bzw. die Bremseinrichtung bzw. die Stativbremse neben der bereits behandelten Bremsbedieneinheit 4 eine Übertragungseinheit 6 (hier nur gestrichelt gezeichnet) und die eigentliche Bremse 7. Die Bremse 7 ist ebenfalls nur schematisch dargestellt. Es kann sich hierbei um einzelne Rollenbremsen an den Rollen 8 des Stativs handeln oder aber um eine Bremse 7, die an geeigneter Stelle angeordnet ist, um die Rollen 8 des Stativs 2 zu bremsen bzw. zu blockieren. Dem Fachmann sind geeignete konkrete Ausgestaltungen einer solchen Bremse 7 bekannt.

Die Übertragungseinheit 6 und/oder die Bremse 7 kann mechanisch, elektronisch oder hydraulisch oder in einer Mischform ausgebildet sein. In der rein mechanischen Ausführungsform wird bspw. durch Herabdrücken der Bremsbedieneinheit 4 ein Zug auf die beispielsweise als Übertragungsseil ausgebildete Übertragungseinheit 6 ausgeübt. Dieser Zug führt zu einem Aufgehen der Bremse 7. Die Rollen 8 sind dann frei beweglich. Bei vorhandener Antriebseinheit 9 können die Rollen 8 zusätzlich unterstützend angetrieben werden, um das Stativ leichter zu verfahren. Sobald die Bremsbedieneinheit 4 nicht mehr herabgedrückt wird, also in ihre Ausgangsposition zurückkehrt, wird auf die als Übertragungsseil ausgebildete Übertragungseinheit 6 kein Zug mehr ausgeübt. Dies führt zu einem Zugehen der Bremse 7 mit der Folge der Blockade der Rollen 8. Hierbei ist es sinnvoll, durch elektronische hydraulische Bremsunterstützung ein abruptes Blockieren der Rollen 8 zu vermeiden und stattdessen die Rollen 8 gleichmäßig aber effektiv abzubremsen.

Alternativ kann durch mechanisches Herabdrücken der Bremsbedieneinheit 4 elektronisch ein Signal ausgelöst werden, das mittels einer elektronischen Übertragungseinheit 6 zu einer Bremse 7 übertragen wird. Die Bremse 7 kann dann elektromechanisch die Bremsung bzw. Blockade der Rollen 8 aufheben, sodass die Rollen 8 verfahren werden können. Auch hier bietet sich die oben beschriebene Unterstützung durch eine Antriebseinheit 9 zum leichteren Verfahren des Stativs 2 an. Sobald die Bremsbedieneinheit 4 nicht mehr herabgedrückt wird, wird die Bremse 7 aktiviert. Dies kann über ein weiteres anderes elektronisches Signal oder aber durch Fehlen des oben erwähnten elektronischen Signals mittels der elektronischen Übertragungseinheit 6 ausgelöst werden. Die Bremse 7 bewirkt dann elektromechanisch eine Bremsung bzw. Blockade der Rollen 8, wobei auch hier wiederrum eine elektronische/hydraulische Bremsunterstützung vorteilhaft sein kann. Konkrete Ausführungsformen einer derartigen Bremsbetätigung sind dem Fachmann ebenfalls bekannt und sollen daher vorliegend nicht näher erläutert werden.

### Bezugszeichenliste

- 1: Mikroskop
- 2: Stativ
- 3: Bremseinrichtung, Totmannbremse
- 4: Bremsbedieneinheit
- 5: Handgriff des Stativs
- 6: Übertragungseinheit
- 7: Bremse
- 8: Rollen des Stativs
- 9: Antriebseinheit

## Patentansprüche

1. Mikroskop (1) mit einem Stativ (2), wobei das Mikroskop als Operationsmikroskop ausgebildet ist, wobei das Stativ (2) eine Bremseinrichtung (3) zur Arretierung des Stativs (2) aufweist, und wobei das Stativ (2) Rollen (8) zum Verfahren des Stativs (2) aufweist,
**dadurch gekennzeichnet, dass**
die Bremseinrichtung als Totmannbremse (3) ausgeführt ist, wobei eine Nichtbetätigung der Totmannbremse (3) durch Aktivierung einer Bremse (7) zum Blockieren wenigstens einer der Rollen (8) das Stativ (2) arretiert.

2. Mikroskop (1) nach Anspruch 1, **dadurch gekennzeichnet, dass** die Totmannbremse (3) über wenigstens eine Bremsbedieneinheit (4) verfügt, durch deren Betätigung ein Lösen der Totmannbremse (3) erfolgt.

3. Mikroskop (1) nach Anspruch 2, **dadurch gekennzeichnet, dass** die Bremsbedieneinheit (4) ein Bedienhebel, Bedienknopf und/oder Bedienschalter ist.

4. Mikroskop (1) nach Anspruch 3, **dadurch gekennzeichnet, dass** die Bremsbedieneinheit (4) ein von Hand bedienbarer Bedienhebel, Bedienknopf und/oder Bedienschalter ist.

5. Mikroskop (1) nach Anspruch 4, **dadurch gekennzeichnet, dass** die Bremsbedieneinheit (4) einen Handgriff (5) des Stativs (2) umfasst.

6. Mikroskop (1) nach Anspruch 4, **dadurch gekennzeichnet, dass** die Bremsbedieneinheit (4) an einem Handgriff (5) des Stativs (2) ausgebildet ist.

7. Mikroskop (1) nach Anspruch 6, **dadurch gekennzeichnet, dass** die Bremsbedieneinheit (4) derart an dem Handgriff (5) des Stativs (2) angeordnet ist, dass die Bremsbedieneinheit (4) und der Handgriff (5) zusammen mit einer Hand betätigt werden können.

8. Mikroskop (1) nach einem der vorangehenden Ansprüche, **dadurch gekennzeichnet, dass** die Totmannbremse (3) neben der Bremsbedieneinheit (4) eine Übertragungseinheit (6) zu einer oder mehreren Bremsen (7) aufweist.

9. Mikroskop (1) nach Anspruch 8, **dadurch gekennzeichnet, dass** die Totmannbremse (3) mechanisch, elektronisch oder hydraulisch oder in einer Mischform ausgebildet ist.

10. Mikroskop (1) nach Anspruch 9, wobei die Totmannbremse (3) eine Stromversorgung aufweist.

11. Mikroskop (1) nach einem der Ansprüche 1 bis 10, **dadurch gekennzeichnet, dass** eine Antriebseinheit (9) zum Antreiben mindestens einer der Rollen (8) vorgesehen ist.

12. Mikroskop (1) nach Anspruch 11, **dadurch gekennzeichnet, dass** die Antriebseinheit (9) derart eingerichtet ist, dass sie die mindestens eine Rolle unterstützend antreibt, wenn ein Lösen der Totmannbremse erfolgt.

## Claims

1. Microscope (1) having a stand (2), the microscope being designed as an surgical microscope, the tripod (2) having a braking device (3) for locking the stand (2), and the stand (2) having rolls (8) for moving the tripod (2),
**characterised in that**
the braking device is designed as a dead man's brake (3), wherein a non-operation of the dead man's brake (3), by activating a brake (7) for blocking at least one of the rolls (8), locks the stand (2).

2. Microscope (1) according to claim 1, **characterized in that** the dead man's brake (3) has at least one brake operating unit (4), the operation of which releases the dead man's brake (3).

3. Microscope (1) according to claim 2, **characterized in that** the brake operating unit (4) is an operating lever, operating button and/or operating switch.

4. Microscope (1) according to claim 3, **characterized in that** the brake operating unit (4) is a manually operable operating lever, operating button and/or operating switch.

5. Microscope (1) according to claim 4, **characterized in that** the brake control unit (4) comprises a handle (5) of the stand (2).

6. Microscope (1) according to Claim 4, **characterized in that** the brake operating unit (4) is formed on a handle (5) of the stand (2).

7. Microscope (1) according to claim 6, **characterized in that** the brake operating unit (4) is arranged on the handle (5) of the stand (2) in such a manner that the brake operating unit (4) and the handle (5) are operable together with one hand.

8. Microscope (1) according to one of the preceding claims, **characterized in that** the dead man's brake (3) has, in addition to the brake operating unit (4), a transmission unit (6) to one or more brakes (7).

9. Microscope (1) according to claim 8, **characterized in that** the dead man's brake (3) is designed mechanically, electronically or hydraulically or in a mixed form.

10. Microscope (1) according to claim 9, wherein the dead man's brake (3) has a power supply.

11. Microscope (1) according to any one of claims 1 to 10, **characterized in that** a driving unit (9) is provided for driving at least one of the rolls (8).

12. Microscope (1) according to claim 11, **characterized in that** the driving unit (9) is configured in such a manner that it drives the at least one roll in a supporting manner when the dead man's brake is released.

## Revendications

1. Microscope (1) comportant un statif (2), le microscope étant conçu comme un microscope opératoire, le statif (2) comportant un dispositif de freinage (3) pour bloquer le trépied (2), et le statif (2) comportant des rouleaux (8) pour déplacer le statif (2),
**caractérisé en ce que**
le dispositif de freinage est conçu comme un frein d'homme mort (3), dans lequel un non actionnement du frein d'homme mort (3) par activation d'un frein (7) pour bloquer au moins l'un des rouleaux (8) bloquant le statif (2).

2. Microscope (1) selon la revendication 1, **caractérisé en ce que** le frein de l'homme mort (3) présente au moins une unité de commande de frein (4), dont l'actionnement libère le frein de l'homme mort (3).

3. Microscope (1) selon la revendication 2, **caractérisé en ce que** l'unité de commande de frein (4) est un levier de commande, un bouton de commande et/ou un interrupteur de commande.

4. Microscope (1) selon la revendication 3, **caractérisé en ce que** l'unité de commande de frein (4) est un levier de commande, un bouton de commande et/ou un interrupteur de commande à actionnement manuel.

5. Microscope (1) selon la revendication 4, **caractérisé en ce que** l'unité de commande de frein (4) comprend une poignée (5) du statif (2).

6. Microscope (1) selon la revendication 4, **caractérisé en ce que** l'unité de commande de frein (4) est formée sur une poignée (5) du statif (2).

7. Microscope (1) selon la revendication 6, **caractérisé en ce que** l'unité de commande de frein (4) est disposée sur la poignée (5) du statif (2) de telle sorte que l'unité de commande de frein (4) et la poignée (5) peuvent être actionnées ensemble à une main.

8. Microscope (1) selon l'une des revendications précédentes, **caractérisé en ce que** le frein de l'homme mort (3) comporte, en plus de l'unité de commande de frein (4), une unité de transmission (6) à un ou plusieurs freins (7).

9. Microscope (1) selon la revendication 8, **caractérisé en ce que** le frein de l'homme mort (3) est conçu mécaniquement, électroniquement ou hydrauliquement ou sous une forme mixte.

10. Microscope (1) selon la revendication 9, le frein de l'homme mort (3) ayant une alimentation électrique.

11. Microscope (1) selon l'une des revendications 1 à 10, **caractérisé en ce qu'**une unité d'entraînement (9) est prévue pour entraîner au moins un des rouleaux (8).

12. Microscope (1) selon la revendication 11, **caractérisé en ce que** l'unité d'entraînement (9) est disposée de telle sorte qu'elle entraîne l'au moins un rouleau d'appui lorsque le frein de l'homme mort est desserré.
